# FASCICULE DE BREVET EUROPEEN

(11) **EP 1 109 742 B1**
(45) Date de publication et mention de la délivrance du brevet: **09.03.2005**
(21) Numéro de dépôt: 00936999.2
(22) Date de dépôt: 02.06.2000
(51) Int. Cl.: C02F 1/02

(54) **APPAREIL DE TRAITEMENT DE L'EAU**
WASSERBEHANDLUNGSVORRICHTUNG
WATER TREATMENT APPARATUS

(30) Priorité: 03.06.1999 FR 9907380
(43) Date de publication de la demande: 27.06.2001
(73) Titulaire: Proteaup, 78630 Orgeval (FR)
(72) Inventeur: LARGUIER, Maurice Gabriel, 94340 Joinville le Pont (FR)
(74) Mandataire: Breesé, Pierre
(86) Numéro de dépôt international: PCT/FR2000/001524
(87) Numéro de publication internationale: WO 2000/075077

(56) Documents cités:
- EP-A- 0 598 652
- DE-A- 19 745 200
- FR-E- 702

## Description

La présente invention concerne un appareil de traitement de l'eau en général, et plus particulièrement un appareil de destruction ou d'éradication des micro-organismes éventuellement pathogènes qui peuvent y être contenus.

On connaît déjà, par exemple du document EP-A- 0,766 647, un appareil de traitement de liquides contenant des matériaux organiques et/ou des micro-organismes, qui comporte une surface sur laquelle le liquide est amené à s'écouler, et qui porte un film de semi-conducteur qui est irradié de telle manière qu'une réaction photochimique soit engagée. Une tension est appliquée au matériau semi-conducteur pour s'assurer que les électrons, libérés par la réaction, soient transportés toin du semi-conducteur. Un tel appareil emploie un film de semi-conducteur formé thermiquement ou à l'aide d'un aerogel. Cet appareil est utilisé pour décomposer les solutions organiques ou pour enlever les micro-organismes de l'eau, par exemple pour purifier des eaux usées contenant des résidus ou des composés organiques et pour en extraire les bactéries pathogènes.

Dans cet appareil connu, outre son coût relativement élevé, l'effet de purification ou de traitement est limité à une zone active, ce qui a pour conséquences de ne pas pouvoir contrôler le développement des micro-organismes en aval de la zone de traitement, et également de ne pas pouvoir assurer une fourniture en continu d'une eau purifiée de tout micro-organisme pouvant être pathogène.

On connaît également du document DE-A-197 45 200 un procédé et un dispositif pour la destruction des agents pathogènes dans les dispositifs d'approvisionnement en eau. Ce document ne décrit ni la structure du dispositif d'échange de chaleur, ni la structure du surchauffeur.

On connaît encore du document FR-321 031 un appareil stérilisateur de technique très ancienne. Pas plus que le document précédent, ce document ne décrit ni la structure du surchauffeur, ni la structure du dispositif de puisage.

On connaît enfin du document EP-A-0 598 652 un appareil de production d'eau stérile chaude ou froide à partir de l'eau du robinet. Il ne décrit ni la structure du dispositif d'échange de chaleur, ni la structure du surchauffeur, ni la structure du dispositif de puisage.

La présente invention se place dans ce contexte et elle a pour objet de proposer un appareil de traitement de l'eau, permettant une purification de cette eau en continu de sorte qu'il soit possible de puiser de l'eau pure en permanence et de façon continue, en étant assuré que cette eau possède le degré de pureté requis, cet appareil devant être de conception simple de manière à être de coût réduit, qui soit facile à mettre en oeuvre même par un personnel peu qualifié.

Dans ce but, la présente invention propose un appareil de traitement de l'eau, comprenant en combinaison, entre un dispositif d'entrée et un dispositif de sortie, un échangeur de chaleur, un surchauffeur et un dispositif de puisage, l'eau circulant successivement dans l'échangeur de chaleur où sa température s'élève, dans le surchauffeur où sa température s'élève encore, dans l'échangeur de chaleur où sa température s'abaisse, et dans le dispositif de puisage.

Selon la présente invention, l'échangeur de chaleur comporte au moins une cuve dans laquelle est disposée au moins une canalisation en serpentin, l'eau provenant du dispositif d'entrée circulant dans la cuve, l'eau provenant du surchauffeur circulant dans la canalisation, et le surchauffeur comporte au moins un corps, dans lequel est formée au moins une canalisation pour la circulation de l'eau à traiter, et au moins un alésage destiné à recevoir au moins une cartouche chauffante.

Selon d'autres caractéristiques de la présente invention,
- il comporte en outre un dispositif de régulation de la température du fluide délivré par le dispositif de puisage,
- le dispositif de régulation de la température du fluide délivré par le dispositif de puisage comporte une surface de contact se présentant sous la forme d'une paroi façonnée sans raccord, sans joint, sans soudure ou autre pièce mécanique intérieure au dispositif,
- le dispositif de puisage comporte un corps traversé par un canal, une résistance électrique étant installée dans le corps à proximité du canal, et un système d'isolation thermique étant disposé autour du corps,
- le dispositif de puisage peut être utilisé séparément et peut comporter au moins une canalisation supplémentaire de distribution d'un deuxième fluide,
- le deuxième fluide est de nature différente,
- le deuxième fluide est de l'eau à une température prédéterminée indépendante de la température de l'eau à la sortie du dispositif d'échange de chaleur,
- le deuxième fluide est de l'eau déminéralisée,
- le dispositif d'échange de chaleur comporte au moins une cuve dans laquelle sont disposées des surfaces spécifiques d'échange de chaleur dont la nature et la disposition confèrent à l'eau en sortie du dispositif d'échange de chaleur des propriétés prédéterminées,
- les surfaces spécifiques d'échange de chaleur sont des plaques,
- le surchauffeur porte la température de l'eau à une valeur supérieure à la température d'ébullition, et
- il est muni de dispositifs de sécurité pour déclencher une procédure automatique d'autodécontamination lors de la mise en service de l'appareil de traitement de l'eau, ou lors de chaque remise en service après un arrêt prolongé d'une durée prédéterminée.

D'autres buts, caractéristiques et avantages de la présente invention apparaîtront clairement de la description qui va maintenant en être faite à titre d'illustration seulement, en référence aux dessins annexés sur lesquels :
- La Figure 1 représente une vue schématique illustrant le principe de l'appareil de traitement de l'eau selon la présente invention;
- La Figure 2 représente une vue en perspective d'un appareil de traitement de l'eau réalisé selon la présente invention, et mettant en application le principe de la Figure 1;
- La Figure 3 représente une vue en perspective, partiellement éclatée, d'un échangeur de chaleur faisant partie de l'appareil de traitement de l'eau représenté sur la Figure 2;
- La Figure 4 représente une vue en perspective, partiellement éclatée, d'un surchauffeur faisant partie de l'appareil de traitement de l'eau représenté sur la Figure 2, et
- La Figure 5 représente une vue en coupe d'un dispositif de puisage selon la présente invention et faisant partie de l'appareil de traitement de l'eau représenté sur la Figure 2.

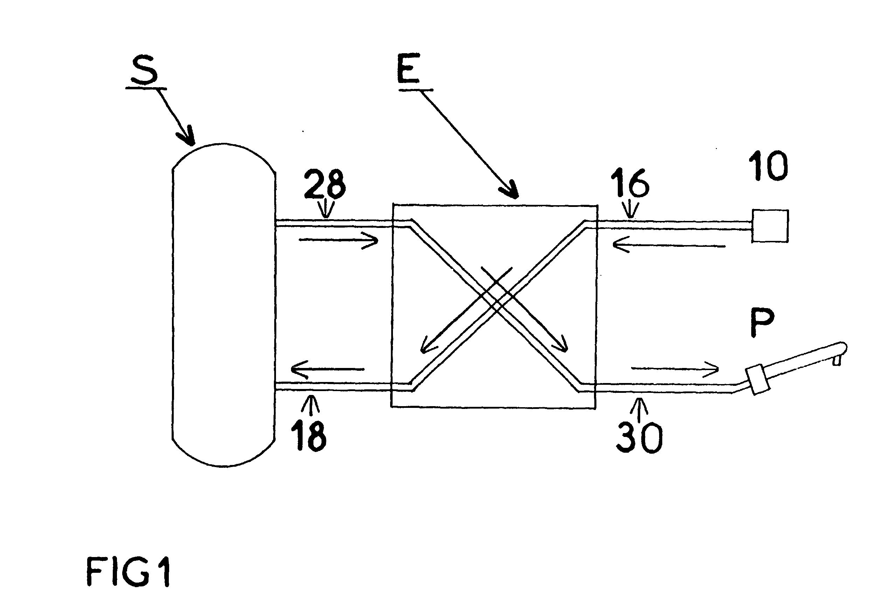

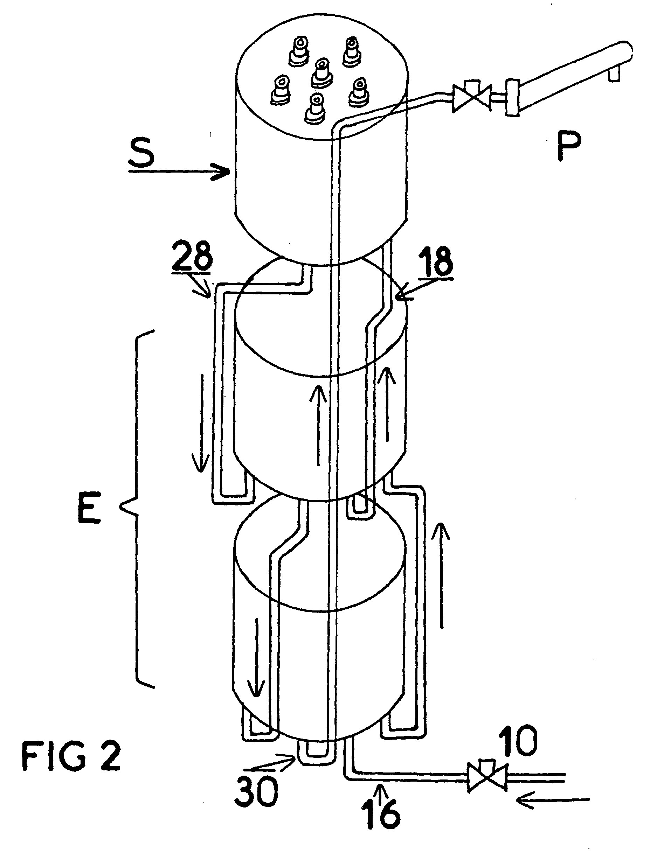

On a représenté sur la Figure 1 un schéma de principe de l'appareil de traitement de l'eau, et sur la Figure 2 une vue en perspective de cet appareil de traitement de l'eau, destiné à en éliminer de façon continue tous les micro-organismes et toutes les bactéries, éventuellement pathogènes, qui pourraient y être présents. Un tel appareil comporte une entrée ou arrivée d'eau 10, destinée par exemple à être raccordée à une canalisation du réseau de distribution d'eau sous pression d'un immeuble, et constituée par exemple par un robinet d'arrêt. L'eau ainsi admise dans l'appareil passe ensuite dans un échangeur de chaleur E, où elle subit une première élévation de température. Cette eau ainsi préchauffée passe ensuite dans un surchauffeur S, où sa température est élevée à plus de 100 °C. En sortie du surchauffeur S, l'eau repasse dans l'échangeur de chaleur E, où elle cède une partie de sa chaleur à l'eau arrivant en amont du surchauffeur. L'eau ainsi refroidie peut ensuite être utilisée, par l'intermédiaire d'un dispositif de puisage P, éventuellement muni d'un robinet de distribution, l'eau issue du dispositif de puisage étant exempte de tout micro-organisme ou de toute bactérie pathogène comme on va le voir par la suite.

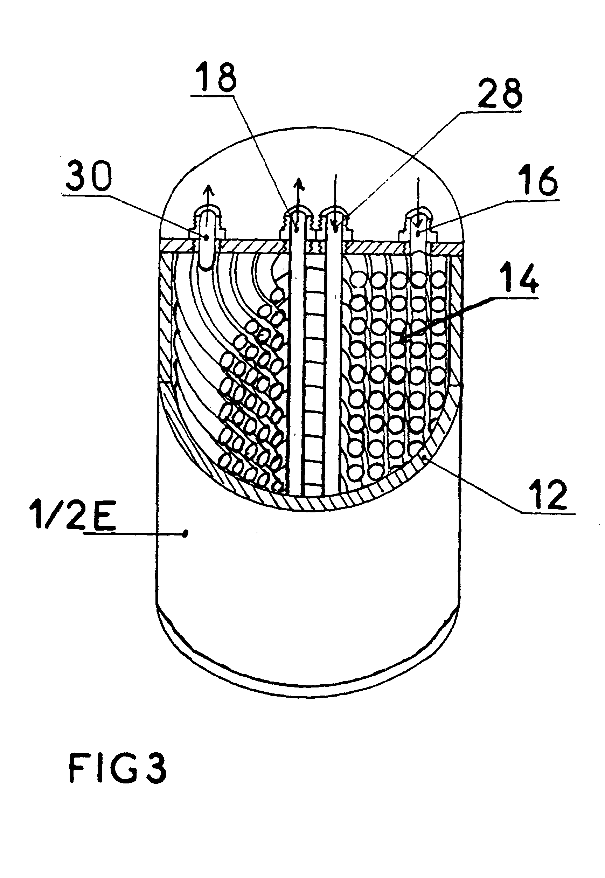

De façon plus précise, l'eau à traiter est admise dans l'échangeur de chaleur, représenté plus en détail sur la Figure 3, après être passée éventuellement par le robinet d'arrêt 10, où elle subit une première élévation de température. Comme on peut le voir sur la Figure 3, l'échangeur de chaleur E est constitué d'une cuve 12, par exemple cylindrique,
dans laquelle est disposée une canalisation 14 en serpentin. En variante, on peut prévoir que plusieurs serpentins soient disposés dans la cuve 12, et soient enroulés de manière coaxiale. L'eau arrivant dans l'appareil de traitement pénètre dans la cuve 12 par une canalisation 16, et elle en ressort par une canalisation 18, après avoir circulé autour du ou des serpentins 14. Dans le ou les serpentins 14 circule l'eau qui provient du surchauffeur S, de sorte que la température de l'eau s'élève entre son entrée par la canalisation 16 et sa sortie par la canalisation 18.

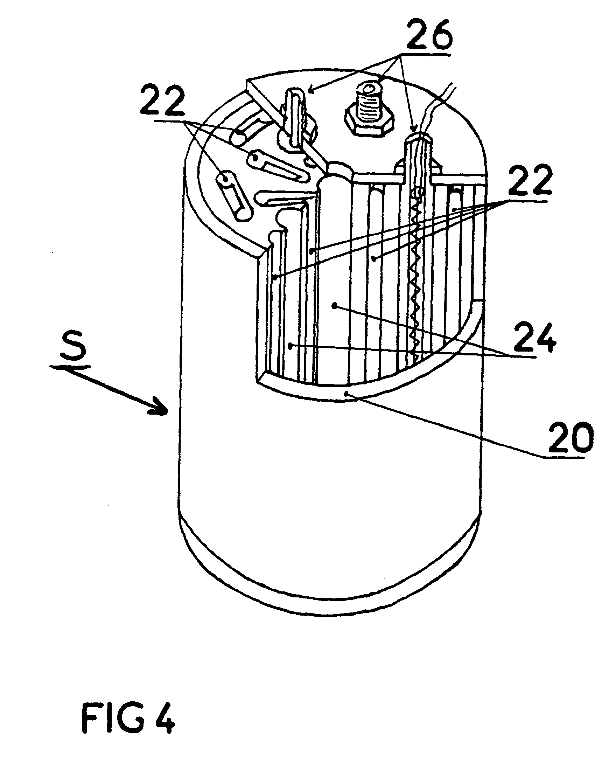

La canalisation 18 en sortie de l'échangeur de chaleur E est reliée à l'entrée du surchauffeur S, représenté plus en détail sur la Figure 4. Le surchauffeur S est constitué d'un corps 20, dans lequel sont formées des canalisations 22 pour la circulation de l'eau à traiter, et dans lequel sont également formés des alésages 24 destinés à recevoir des cartouches chauffantes 26, par exemple des résistances électriques. Dans le surchauffeur S, l'eau est ainsi portée à une température comprise entre 90 °C et 120 °C, et de préférence supérieure à 100 °C, c'est à dire supérieure à la température d'ébullition à la pression ambiante, de sorte qu'elle subit un choc thermique, éliminant tous les micro-organismes, germes, bactéries ou microbes qui pourraient y être présents.

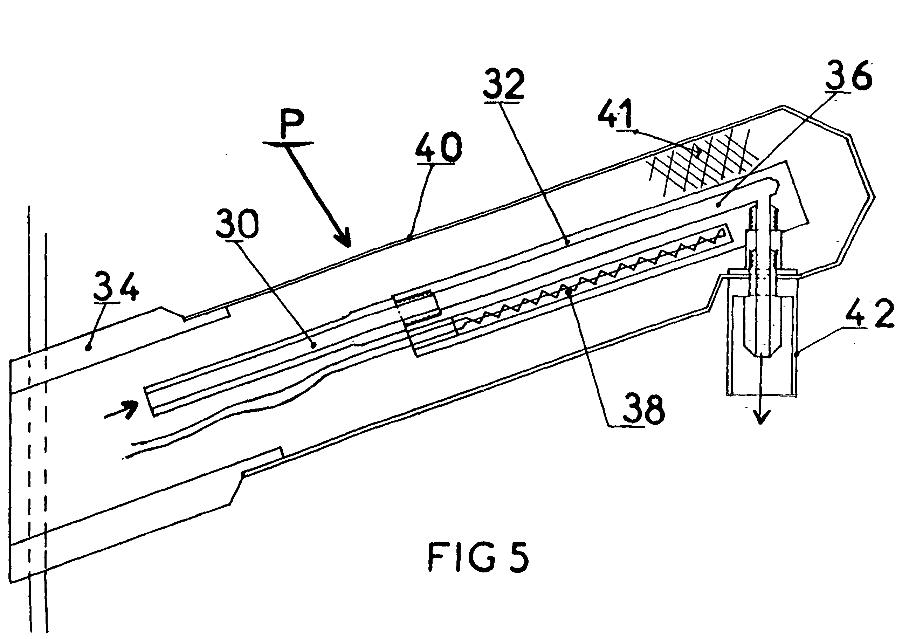

L'eau ainsi traitée ressort du surchauffeur S par une canalisation 28 pour retourner dans l'échangeur de chaleur E, et circuler dans le ou les serpentins 14 de ce dernier. Comme on l'a vu plus haut, la paroi externe du ou des serpentins 14 est en contact avec l'eau à traiter pénétrant dans l'appareil, et donc à température inférieure. L'eau traitée est ainsi refroidie par l'eau à traiter, et elle ressort de l'échangeur de chaleur E par une canalisation 30, menant au dispositif de puisage P, représenté plus en détail sur la Figure 5.

Le dispositif de puisage P est constitué d'un corps métallique 32, par exemple cylindrique, comportant un support 34, et traversé par un canal 36 dont un des orifices est ouvert à l'atmosphère pour pouvoir effectuer le puisage. Une résistance électrique 38 est installée dans le corps 32, à proximité du canal 36. Un système d'isolation thermique 40 est disposé dans le corps 32, et une collerette de protection 42 est disposée au voisinage immédiat de l'extrémité ouverte vers l'extérieur du canal 36, de manière à éviter tout contact avec l'extrémité du canal 36. Un robinet de distribution (non représenté) peut avantageusement être disposé sur la canalisation 30, en aval de l'échangeur de chaleur E et en amont du dispositif de puisage P.

De manière avantageuse, la résistance électrique 38 fait partie d'un dispositif de régulation de la température du fluide délivré par le dispositif de puisage P. Un tel dispositif peut comporter une surface de contact avec le fluide traité se présentant sous la forme d'une paroi façonnée sans raccord, sans joint, sans soudure ou autre pièce mécanique à l'intérieur du dispositif.

A la lecture de la description qui précède, on aura compris comment la présente invention peut être mise en oeuvre. Lorsque, le robinet d'arrêt 10 étant ouvert, un utilisateur désire disposer d'eau exempte de micro-organismes, il ouvre le robinet de distribution disposé en amont du dispositif de puisage P. De l'eau peut ainsi être admise dans l'appareil de traitement de l'eau que l'on vient de décrire. Cette eau passe successivement dans l'échangeur de chaleur E, où elle subit une première élévation de température jusqu'à environ 50°C, dans le surchauffeur S où elle subit un choc thermique compris entre 90 °C et 120 °C, puis à nouveau dans l'échangeur de chaleur E où sa température est abaissée jusqu'à environ 50 °C, puis enfin dans le dispositif de puisage P d'où elle peut être recueillie par l'utilisateur, en étant débarrassée de tout micro-organisme.

Après s'être ainsi servi en eau traitée, l'utilisateur referme le robinet de distribution. L'eau cesse donc de circuler dans l'appareil de traitement de l'eau. L'eau contenue dans le surchauffeur S est cependant maintenue en température (entre 90 °C et 120 °C) afin d'éviter toute prolifération de germes ou de micro-organismes. De plus, ce maintien en température de l'eau contenue dans le surchauffeur S permet d'éviter toute attente lors d'une demande ultérieure de puisage d'eau traitée. De même, l'eau contenue dans le dispositif de puisage P est maintenue, grâce au système d'isolation thermique 40 et à la résistance chauffante 38, à une température d'environ 90 °C de manière à éviter toute pénétration de micro-organismes ou de germes en provenance de l'extérieur, ou toute prolifération de ces agents infectieux sur toutes les surfaces de l'appareil pouvant être au contact de l'eau.

L'appareil de traitement de l'eau selon la présente invention peut avantageusement être muni de dispositifs de sécurité pouvant entrer en action à différentes occasions. Une procédure automatique d'autodécontamination peut être prévue lors de la mise en service de l'appareil de traitement de l'eau selon la présente invention, ou lors de chaque remise en service après un arrêt prolongé d'une durée prédéterminée. Une telle procédure peut par exemple consister en une mise sous tension de l'appareil de manière à atteindre un seuil de température, typiquement de l'ordre de 120 °C, une mise en situation dynamique à très faible débit pendant environ 12 minutes de sorte que l'ensemble des canalisations en contact avec l'eau, ait été porté à une température minimale de 120 °C, après quoi l'utilisation de l'appareil de traitement de l'eau est autorisé.

En cas de dysfonctionnement électrique, les dispositifs de sécurité peuvent inhiber l'appareil de traitement de l'eau et en interdire l'utilisation, la remise en service ne pouvant intervenir qu'après un diagnostic du dysfonctionnement et une intervention sur l'appareil. En cas de dysfonctionnement hydraulique, par exemple une fuite dans l'une des canalisations constituant l'appareil, les dispositifs de sécurité, par exemple constitués de détecteurs de pression à sécurité positive, peuvent de la même manière inhiber l'appareil de traitement de l'eau et en interdire l'utilisation, l'eau qui serait fournie dans ces conditions pouvant ne plus être exempte de micro-organismes ou de germes pathogènes.

On a donc bien réalisé un appareil de traitement de l'eau, permettant une purification de cette eau de manière continue, de sorte qu'il est possible de puiser de l'eau traitée en permanence, en étant assuré que cette eau possède le degré de pureté requis. Cet appareil est de conception particulièrement simple de sorte qu'il ne présente qu'un coût réduit, et il est facile à mettre en oeuvre même par un personnel peu qualifié. Il constitue également un dispositif thermique de lutte contre toute contamination microbiologique intérieure et extérieure à l'orifice de sortie de l'eau traitée.

Bien entendu, la présente invention n'est pas limitée au mode de réalisation qui vient d'être décrit, mais elle est au contraire susceptible de recevoir de nombreuses modifications qui apparaîtront à l'homme du métier sans sortir de son cadre. C'est ainsi par exemple que l'on pourra prévoir que le dispositif de puisage comporte plusieurs canaux et orifices de distribution, pour permettre une distribution d'eau indépendante à chacun des orifices de distribution, les qualités de l'eau à ces différents orifices de distribution pouvant être différentes par l'utilisation de résistances chauffantes de puissances différentes. De même, le robinet de distribution en amont du dispositif de puisage pourra être constitué d'une valve fonctionnant en tout ou rien, la commande de cette valve ou de ce robinet pouvant être manuelle, une commande au pied, ou par une cellule photoélectrique. Enfin, l'échangeur de chaleur et le surchauffeur pourront comprendre un, deux ou plusieurs modules selon la température désirée pour l'eau à leur sortie.

## Revendications

1. Dispositif de puisage (P), **caractérisé en ce qu'**il comporte un corps (32) traversé par un canal (36), une résistance électrique (38) installée dans le corps (32) à proximité du canal (36), et un système d'isolation thermique (40) disposé autour du corps (32).

2. Dispositif de puisage (P) selon la revendication 1, **caractérisé en ce qu'**il comporte au moins une canalisation supplémentaire de distribution d'un deuxième fluide.

3. Dispositif de puisage (P) selon l'une quelconque des revendications 1 ou 2, **caractérisé en ce que** le deuxième fluide est de nature différente.

4. Dispositif de puisage (P) selon l'une quelconque des revendications 1 à 3, **caractérisé en ce que** le deuxième fluide est de l'eau à une température prédéterminée indépendante de la température de l'eau à la sortie du dispositif d'échange de chaleur.

5. Dispositif de puisage (P) selon l'une quelconque des revendication 1 à 4, **caractérisé en ce que** le deuxième fluide est de l'eau déminéralisée.

6. Appareil de traitement de l'eau, comprenant en combinaison, entre un dispositif d'entrée (10) et un dispositif de sortie (P), un échangeur de chaleur (E), un surchauffeur (S) et un dispositif de puisage (P), l'eau circulant successivement dans l'échangeur de chaleur (E) où sa température s'élève, dans le surchauffeur (S) où sa température s'élève encore, dans l'échangeur de chaleur (E) où sa température s'abaisse, et dans le dispositif de puisage (P), **caractérisé en ce que** l'échangeur de chaleur (E) comporte au moins une cuve (12) dans laquelle est disposée au moins une canalisation (14) en serpentin, l'eau provenant du dispositif d'entrée (10) circulant dans la cuve (12), l'eau provenant du surchauffeur circulant dans la canalisation (14), et **en ce que** le surchauffeur (S) comporte au moins un corps (20), dans lequel est formée au moins une canalisation (22) pour la circulation de l'eau à traiter, et au moins un alésage (24) destiné à recevoir au moins une cartouche chauffante (26) et **en ce que** le dispositif de puisage est tel que décrit dans l'une quelconque des revendications 1 à 5.

7. Appareil de traitement de l'eau selon la revendication 6, **caractérisé en ce qu'**il comporte en outre un dispositif de régulation de la température du fluide délivré par le dispositif de puisage (P).

8. Appareil de traitement de l'eau selon l'une quelconque des revendications 6 ou 7, **caractérisé en ce que** le dispositif de régulation de la température du fluide délivré par le dispositif de puisage (P) comporte une surface de contact se présentant sous la forme d'une paroi façonnée sans raccord, sans joint, sans soudure ou autre pièce mécanique intérieure au dispositif.

9. Appareil de traitement de l'eau selon l'une quelconque des revendications 6 à 8, **caractérisé en ce que** le dispositif d'échange de chaleur (E) comporte au moins une cuve (12) dans laquelle sont disposées des surfaces spécifiques d'échange de chaleur dont la nature et la disposition confèrent à l'eau en sortie du dispositif d'échange de chaleur (E) des propriétés prédéterminées.

10. Appareil de traitement de l'eau selon la revendication 9, **caractérisé en ce que** les surfaces spécifiques d'échange de chaleur sont des plaques.

11. Appareil de traitement de l'eau selon l'une quelconque des revendications 6 à 10, **caractérisé en ce que** la température de l'eau dans le surchauffeur (S) est à une valeur supérieure à la température d'ébullition.

12. Appareil de traitement de l'eau selon l'une quelconque des revendications 6 à 11, **caractérisé en ce qu'**il est muni de dispositifs de sécurité pour déclencher une procédure automatique d'autodécontamination lors de la mise en service de l'appareil de traitement de l'eau, ou lors de chaque remise en service après un arrêt prolongé d'une durée prédéterminée.

## Patentansprüche

1. Schöpfvorrichtung (P) **dadurch gekennzeichnet, dass** sie einen Körper (32) umfasst, der durchquert wird von einem Kanal (36), einem elektrischen Widerstand (38), der in dem Körper (32) in unmittelbarer Nähe vom Kanal (36) installiert ist, und ein thermisches Isolationssystem (40), das um den Körper (32) herum angeordnet ist.

2. Schöpfvorrichtung (P) nach Anspruch 1, **dadurch gekennzeichnet, dass** er mindestens eine weitere Rohrleitung zur Verteilung eines zweiten Fluids umfasst.

3. Schöpfvorrichtung (P) nach einem der vorgehenden Ansprüche 1 oder 2, **dadurch gekennzeichnet, dass** das zweite Fluid anderer Natur ist.

4. Schöpfvorrichtung (P) nach einem der vorgehenden Ansprüche 1 bis 3, **dadurch gekennzeichnet, dass** das zweite Fluid Wasser ist, dessen Temperatur unabhängig von der Temperatur des Wassers am Ausgang von der Wärmeaustauschvorrichtung vorbestimmt ist.

5. Schöpfvorrichtung (P) nach einem der vorgehenden Ansprüche 1 bis 4, **dadurch gekennzeichnet, dass** das zweite Fluid demineralisiertes Wasser ist.

6. Gerät zur Wasserbehandlung, das in Kombinierung zwischen einer Eingangsvorrichtung (10) und einer Ausgangsvorrichtung (P) einen Wärmeaustauscher (E), einen Überhitzer (S) und eine Schöpfvorrichtung (P) umfasst, wobei das Wasser nacheinander im Wärmeaustauscher (E), in dem seine Temperatur steigt, im Überhitzer (S), in dem seine Temperatur weiter steigt, im Wärmeaustauscher (E), in dem seine Temperatur sinkt, und in der Schöpfvorrichtung (P) zirkuliert, **dadurch gekennzeichnet, dass** der Wärmeaustauscher (E) mindestens einen Behälter (12) umfasst, in dem mindestens eine Rohrleitung (14) serpentinartig angeordnet ist, wobei das aus der Eingangsvorrichtung (10) stammende Wasser im Behälter (12) zirkuliert, wobei das aus dem Überhitzer stammende Wasser in der Rohrleitung (14) zirkuliert, und **dadurch gekennzeichnet, dass** der Überhitzer (S) mindestens einen Körper (20) umfasst, in dem mindestens eine Rohrleitung (22) geformt ist für die Zirkulierung des zu behandelnden Wassers, und mindestens eine Bohrung (24), die dazu dient, mindestens eine Heizpatrone (26) aufzunehmen, und **dadurch gekennzeichnet, dass** die Schöpfvorrichtung ihrer Beschreibung in einer der Ansprüche 1 bis 5 entspricht.

7. Gerät zur Wasserbehandlung nach Anspruch 6, **dadurch gekennzeichnet, dass** es weiterhin eine Regelvorrichtung für die Temperatur des Fluids umfasst, das von der Schöpfvorrichtung (P) geliefert wird.

8. Gerät zur Wasserbehandlung nach einem der vorhergehenden Ansprüche 6 oder 7, **dadurch gekennzeichnet, dass** die Regelvorrichtung für die Temperatur des Fluids, das von der Schöpfvorrichtung (P) geliefert wird, eine Kontaktfläche umfasst, die die Form einer ohne Verbindung, ohne Fuge, ohne Schweißnaht oder jedem anderen mechanischen Innenteil der Vorrichtung gestalteten Wand aufweist.

9. Gerät zur Wasserbehandlung nach einem der vorhergehenden Ansprüche 6 bis 8, **dadurch gekennzeichnet, dass** die Vorrichtung für den Wärmeaustausch (E) mindestens einen Behälter (12) umfasst, in dem spezifische Oberflächen für den Wärmeaustausch angeordnet sind, deren Natur und Anordnung dem Wasser am Ausgang der Vorrichtung für den Wärmeaustausch (E) bestimmte Eigenschaften verleihen.

10. Gerät zur Wasserbehandlung nach Anspruch 9, **dadurch gekennzeichnet, dass** es sich bei den spezifischen Oberflächen für den Wärmeaustausch um Platten handelt.

11. Gerät zur Wasserbehandlung nach einem der vorhergehenden Ansprüche 6 bis 10, **dadurch gekennzeichnet, dass** die Temperatur des Wassers im Überhitzter (S) um einen Wert über der Siedtemperatur liegt.

12. Gerät zur Wasserbehandlung nach einem der vorhergehenden Ansprüche 6 bis 11, **dadurch gekennzeichnet, dass** es mit Sicherheitsvorrichtungen ausgerüstet ist, um ein automatisches Verfahren der Selbstdekontamination auszulösen und dies bei der Inbetriebnahme vom Gerät zur Wasserbehandlung oder bei jeder neuen Inbetriebnahme nach einem längeren Stopp zu einer vorbestimmten Dauer.

## Claims

1. Device for drawing up (P), **characterised in that** it comprises a body (32) run through by a canal (36), an electric resistance (38) installed in the body (32) close to the canal (36) and a system of thermal insulation (40) placed around the body (32).

2. Device for drawing up (P) according to claim 1, **characterised in that** it comprises at least an additional distribution pipe for a second fluid.

3. Device for drawing up (P) according to any one of claims 1 or 2, **characterised in that** the second fluid is of a different nature.

4. Device for drawing up (P) according to in any one of claims 1 to 3, **characterised in that** the second fluid is water at a pre-set temperature independent of the water temperature at the outlet of the heat exchanger.

5. Device for extracting (P) set forth in any one of claims 1 to 4, **characterised in that** the second fluid is de-mineralised water.

6. Water purifier, comprising in combination, between an inlet device (10) and an outlet device (P), a heat exchanger (E), a super-heater (S) and a drawing-up device (P), the water successively circulating in the heat exchanger (E) where its temperature rises, in the super-heater (S) where its temperature continues to rise, in the heat exchanger (E) where its temperature drops, and in the drawing-up device (P), **characterised in that** the heat exchanger (E) comprises at least a reservoir (12) in which at least a coil pipe (14) is inserted, the water issuing from the inlet device (10) circulating in the reservoir (12), the water issuing from the super-heater circulating in the pipe (14), and **in that** the super-heater (S) comprises at least a body (20), in which at least one pipe (22) is installed to circulate the purified water, and at least one bore (24) intended to receive at least one cartridge heater (26) and **in that** the drawing-up device is as described in any one of claims 1 to 5.

7. Water purifier according to claim 6, **characterised in that** it further comprises a temperature control for the fluid issuing from the drawing-up device (P).

8. Water purifier according to any one of claims 6 or 7, **characterised in that** the temperature control for the fluid issuing from the drawing-up device (P) comprises a contact surface in the form of a shaped wall without connections, joints, welds or any other mechanical part inside the device.

9. Water purifier according to any one of claims 6 to 8, **characterised in that** the heat exchanger (E) comprises at least a reservoir (12) in which specific heat exchanging surfaces are inserted whose nature and disposition impart, at the outlet of the heat exchanger (E), pre-set properties.

10. Water purifier according to claim 9, **characterised in that** the specific surfaces of the heat exchanger are plates.

11. Water purifier according to any one of claims 6 to 10, **characterised in that** the temperature of the water in the super-heater (S) is greater than boiling point.

12. Water purifier according to any one of claims 6 to 11, **characterised in that** it is equipped with a safety device to actuate an automatic self-decontamination procedure upon commissioning the water purifier, or upon each re-commissioning following a pre-set period of extended shutdown.
